(19) Europäisches Patentamt

European Patent Office

Office européen des brevets

(11) **EP 3 387 986 A1**

(12) **EUROPEAN PATENT APPLICATION**
published in accordance with Art. 153(4) EPC

(43) Date of publication:
**17.10.2018 Bulletin 2018/42**

(21) Application number: **16873452.3**

(22) Date of filing: **08.12.2016**

(51) Int Cl.:
**A61B 5/00** *(2006.01)*    **A61B 5/053** *(2006.01)*

(86) International application number:
**PCT/RU2016/000857**

(87) International publication number:
**WO 2017/099636 (15.06.2017 Gazette 2017/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **10.12.2015 RU 2015153029**

(71) Applicant: **Healbe Corporation**
**Redwood City, CA 94063 (US)**

(72) Inventors:
• **SOLUNIN, Anatoly Aleksandrovich**
**St.Petersburg 199397 (RU)**

• **SOKOLOV, Evgeny Lvovich**
**Leningradskaya oblast**
**g. Gatchina 188307 (RU)**
• **KOLCHIN, Aleksandr Vladimirovich**
**St.Petersburg 198328 (RU)**
• **RUBIN, Mikhail Semenovich**
**St.Petersburg 191119 (RU)**
• **KOLONITSKY, Dmitry Ivanovich**
**St.Petersburg 191123 (RU)**
• **CHECHIK, Andrey Anatol'evich**
**St.Petersburg 190000 (RU)**
• **MISJUCHENKO, Igor Leonidovich**
**St.Petersburg 197371 (RU)**

(74) Representative: **Reichert & Lindner**
**Partnerschaft Patentanwälte**
**Bismarckplatz 8**
**93047 Regensburg (DE)**

(54) **METHOD FOR DETERMINING WATER DEFICIENCY IN A PERSON'S BODY**

(57)    Determining a state of water deficiency in a human body during daily life activities is done by measuring an impedance of a portion of the human body at a low frequency and at a high frequency. An amount of fluid in the body tissues in the volume under study is assessed based on the impedance at the high frequency. A current amount of extracellular fluid in the tissues in the volume is assessed based on the impedance at the low frequency. A reference value of the assessed amount of extracellular fluid in the tissues is preselected at the start of measurements. A correction value is determined based on the changes in blood amount in the volume at the current measurement time. A corrected assessment value is the determined. Using the corrected assessment value, the onset of water deficiency is determined.

FIG. 5

EP 3 387 986 A1

## Description

## FIELD OF THE INVENTION

The invention relates to diagnostics, in particular, to determining a state of water deficiency in a human body during daily life activities by measuring an impedance value of a portion of the human body.

## BACKGROUND OF THE INVENTION

The authors are unaware of other methods of determining the need to replenish the supply of water in a human body during daily life activities by measuring the impedance of a portion of the human body.

Various methods are known to be used for measuring a balance and distribution of water in a human body, mainly for medical purposes.

For example, US Pat. No. 5,469,859 published on November 28, 1995 (IPC A61B 5/085) discloses a method for determining basic cardiorespiratory parameters of a human body. The parameters are determined by applying electrodes to the patient's hands and feet to measure an amount of extracellular fluid and its balance in the entire patient's body. The measurements are made for medical purposes. The above patent does not teach determining a state of water deficiency in a human body during daily life activities.

RU Pat. No. 2314750 published on 20.01.2008 (IPC A61B 5/05) teaches a method for system-related evaluation of blood and fluid dynamics in a human body. According to that method, electrodes are applied to patient's head and extremities for measuring the impedance of various portions of the human body. Slow and violent changes in the impedance are registered and blood and fluid dynamics in peripheral parts and the entire human body is calculating based on those measurements. The method is intended for medical purposes, including monitoring of patient's health condition and selecting medical treatment methods.

US Pat. No. 8,406,865 published on March 26, 2013 (IPC A61B5/00) discloses a system for determining physiological parameters of a human body by measuring an impedance of human body portions via applied electrodes and consequent data processing, which is the closest to the present invention. The primary object of this invention is to determine cardiac and respiratory performance based on impedance measurements. The system enables determination of a water fraction in the lean body mass (i.e. total body mass minus the mass of body fat), a total volume of extracellular fluid, as well as a fluid balance index in the entire patient's body. The system is intended for medical use and does not provide for determining a state of water deficiency in a human body during daily life activities.

A limitation common to those methods is that they are intended for medical purposes only and provide for determining an amount of bodily fluid based on impedance measurements of different portions of a human body.

Since the above methods are primarily used for making measurements in a hospital environment, when the patient is in a quiescent state, they are not applicable for making measurements in everyday life, during work, sports activities, etc.

The applicants are unaware of any methods allowing for determining a state of water deficiency in a human body during daily life activities to warn of the need to replenish water supplies in the body.

## SUMMARY OF THE INVENTION

The technical result of the claimed invention is to determine a state of water deficiency in a human body during daily life activities by measuring an impedance value of a portion of a human body. The above technical result is achieved by assessing a fluid amount in the portion of the human body under study while taking into account changes in the amount of blood in the same body portion caused by a redistribution of fluids in various body portions due to temporary or accidental causes during daily life activities.

The above mentioned technical result is achieved as follows.

The method for detecting water deficiency of in a human body involves the following steps:
Measuring an impedance value of a portion of the human body at a low frequency and at a high frequency. Assessing an amount of fluid in body tissues in a volume under study based on the impedance value measured at the high frequency, at a current measurement time. Assessing an amount of extracellular fluid in the volume under study at the current measurement time based on the impedance value measured at the low frequency. Preselecting a reference assessment value of an amount of extracellular fluid in the volume under study at a reference measurement time. Next, a correction value is determined associated with a change of a blood amount in the volume under study at the current measurement time. Further, determining a corrected assessment value of the amount of fluid in the body tissues in the volume under study, wherein the corrected assessment value is associated with the change of the blood amount in the volume under study at the current measurement time. Using the corrected assessment value of the fluid amount in the volume under study to make a conclusion regarding an onset of water deficiency in the human body.

As known in the art, impedance measurements at a low frequency allow for assessment of an amount of extracellular fluid in a volume under study, whereas impedance measurements at a high frequency allow for assessment of a total amount of fluid in volume under study. The method of the invention allows to make a conclusion regarding an onset of a state of water deficiency in a human body by taking into account a change of a blood amount in the volume under study at the current measurement time. The method allows to eliminate the influence of the change in blood amount in a given body portion caused, for example, by physical exercise or other temporary or random factors on the measurement results, and to make a corrected assessment of the amount of fluid in the body tissues in the volume under study. The latter allows to make a conclusion regarding an onset of water deficiency in the human body.

In a particular embodiment, the impedance values of the portion of the human body are measured using spaced-apart electrodes attached to the human body.

In a specific embodiment, a hematocrit value is taken into consideration for determining the corrected assessment value of the fluid amount in the volume under study associated with change of blood amount in said volume.

In particular, the conclusion regarding the onset of the state of water deficiency in the human body is made based on a maximum value of corrected assessment value of the fluid amount in the body tissues in the volume under study associated with the change of the blood amount in said volume at the current measurement time.

In particular, the impedance values of the portion of the human body at the low and high frequencies are corrected using the readings of a human body temperature sensor.

In addition, the reference measurement time is preselected succeeding a termination of a transition time, which is necessary to adapt the human body to measurement sensors.

## BRIEF DESCRIPTION OF THE DRAWINGS

The method for detecting water deficiency in human body is illustrated by the following drawings:

Fig. 1 shows time dependencies of impedance values measured at different frequencies.
Fig. 2 shows time dependencies of measured impedance values after median filtering with the use of a median filter.
Fig. 3 shows time dependencies of conductivity, which correspond to the amount of fluid in the body tissues in the volume under study; and to the amount of extracellular fluid in the body tissues in said volume.
Fig. 4 shows time dependencies of conductivity at different frequencies, which correspond to the amount of intra- and extracellular fluid in the body tissues in the volume under study, as well as the conductivity, which corresponds to the amount of fluid in the body tissues of said volume minus the change in the blood amount at the current measurement time.
Fig. 5 shows time dependence of conductivity that corresponds to the amount of fluid in the body tissues in the volume under study minus the change in the blood amount since the reference measurement time, as well as the time dependence of conductivity value considered as a threshold for the water deficiency state.

## DETAILED DESCRIPTION OF THE PREFERRED EMBODIMENTS

The method for detecting water deficiency in a human body is implemented as follows.

First, electrodes are attached to the human body. In particular, attachment of two electrodes to a portion of a body is sufficient. Tests have demonstrated that this method provides for determining of an onset of a state of water deficiency in the human body with electrodes attached, for example, to a person's wrist, though this part of the body is not the most suitable for implementing the method. However, the sensor readings of impedance values $Z_i^0$ at a low frequency $f_0$ as well as readings $Z_i^3$ at a high frequency $f_3$ for a given small portion of the human body enable the determination of the water deficiency onset in the body. The most efficient sampling rate for measuring impedance values at different frequencies is found to be about 1 to 4 times per minute.

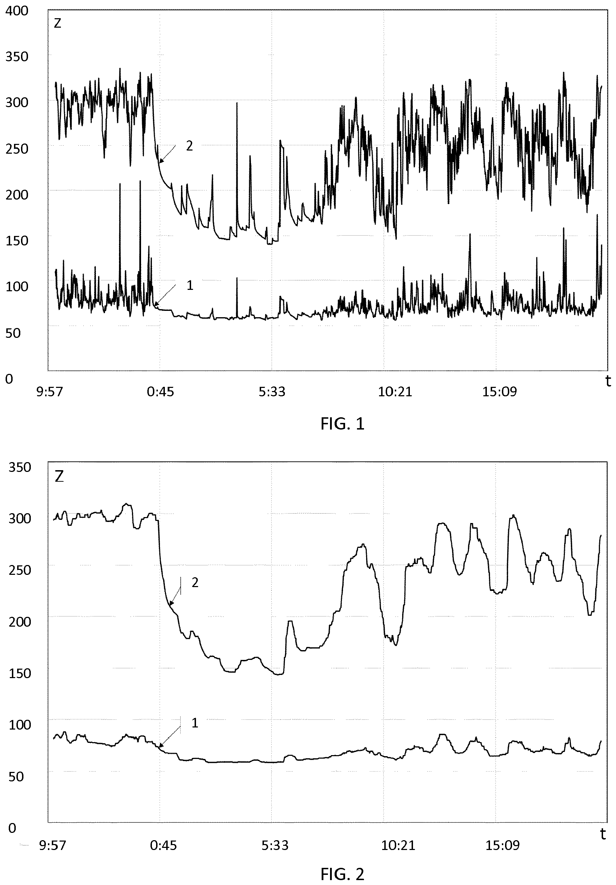

Fig. 1 shows the time dependencies of measured impedance values $Z_{init}^3$ at high frequency f3 (curve 1) and $Z_{init}^0$ at low frequency f0 (curve 2).

To avoid any interference, it is beneficial to use filtration of measured impedance values. Various types of filtration are suitable for this purpose, with the so-called "median filter" applied in this specific case.

Fig. 2 shows the time dependencies of impedance values measured after they were processed using a median filter: $Z_{init}^3$ at high frequency f3 (curve 1) and $Z_{init}^0$ at low frequency f0 (curve 2). Fig. 2 shows that the filtered impedance values contain practically no outliers caused by random factors.

The method can be implemented ignoring the human body temperature. However, the accuracy of the method increases when the body temperature is taken into account. The impedance values $Z_i^0$ measured at low frequency f0 and $Z_i^3$ at high frequency f3 are corrected to take into account the temperature sensor readings by using the following dependencies:

$$Z_{i\,corr}^0 = Z_i^0 \times \left(1 + k_T \times (T_i - T_0)\right),$$

$$Z_{i\,corr}^3 = Z_i^3 \times \left(1 + k_T \times (T_i - T_0)\right),$$

where:

$Z_i^0$ and $Z_i^3$ are instantaneous impedance values at frequency f0 and at frequency f3;

$Z_{i\,corr}^0$ and $Z_{i\,corr}^3$ are instantaneous corrected impedance values at frequency f0 and at frequency f3;

$T_i$ is an instantaneous value of the human body temperature in the impedance measurement area;

$T_0$ is a reference value of the human body temperature in the impedance measurement area;

$k_T$ is a temperature resistance coefficient of the tissue, through which a measuring current passes used for measuring impedance values.

The temperature value at a start of measurements may be selected as the reference value $T_0$.

Based on the value of the impedance measured at high frequency f3, an assessment value of conductivity $y_i^{sum}$ is acquired, which is proportional to the amount of fluid in the body tissues in the volume under study at the current measurement time:

$$y_i^{sum} = 1/Z_{i\,corr}^3.$$

Based on the value of the impedance measured at low frequency $f_0$, an assessment value of conductivity $y_i^{out}$ is acquired, which is proportional to the amount of extracellular fluid in the body tissues in the volume under study at the current measurement time:

$$y_i^{out} = 1/Z_{i\,corr}^0.$$

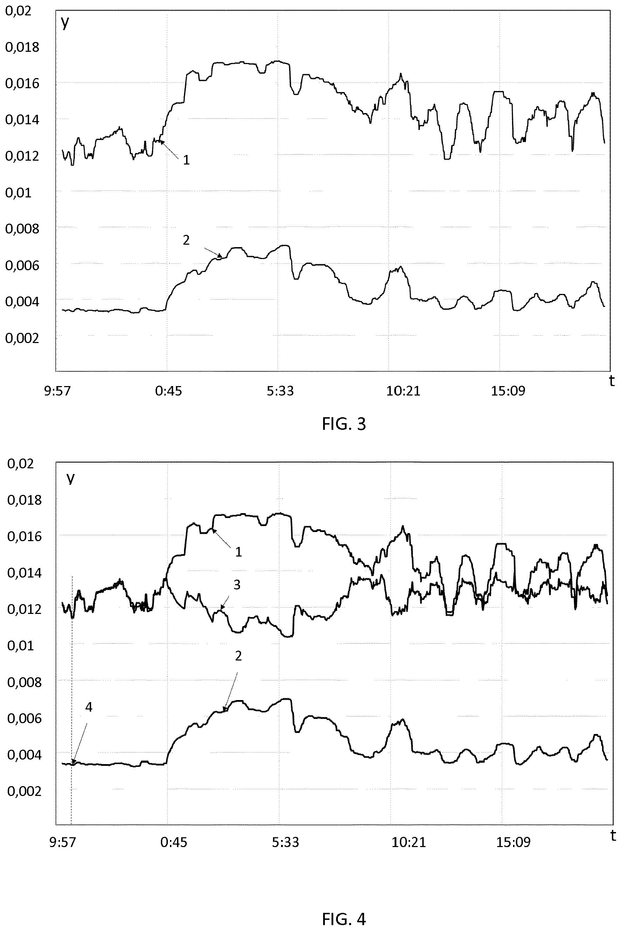

The conductivity characterizing $y_i^{sum}$ (curve 1) - at high frequency f3, which corresponds to the amount of fluid in the body tissues in the volume under study, and $y_i^{out}$ (curve 2) - at low frequency f0, which corresponds to the amount of extracellular fluid in the body tissues in the volume under study are shown in Fig. 3.

At the start of measurements a reference value $y_{base}^{out}$ is preselected, which is proportional to the amount of extracellular fluid in the volume under study at a reference measurement time.

The reference measurement time $t_{base}$ is preselected succeeding a termination of a transition time, necessary to adapt the human body to measurement sensors after they are applied.

Next, value $y_{base}^{out}$ is used to determine the value of correction $\Delta_i^{bl}$ that takes into account the change in the amount of blood in the volume under study at the current measurement time:

$$\Delta_i^{bl} = (y_i^{out} - y_{base}^{out})/(1 - h),$$

where:

h is the hematocrit, i.e. the ratio of the volume of erythrocytes constituting about 99% of blood cells, to the volume of blood plasma; the hematocrit value for females may be considered to be 0.40, whereas for males it may be considered to be 0.46.

Next, a corrected value $y_i'^{sum}$ is found, which is proportional to the amount of fluid in the body tissues in the volume under study and is associated with the change of blood amount in the body tissues in said volume at the current measurement time:

$$y_i'^{sum} = y_i^{sum} - \Delta_i^{bl},$$

where: $y_i^{sum}$ - is the assessed conductivity proportional to the current amount of fluid in the body tissues in the volume under study.

Taking into account changes in the amount of blood in the body tissues in the volume under study enables assessment of both intra- and extracellular fluid amounts in said volume.

The time dependency of conductivity that corresponds to the amount of fluid in the body tissues in the volume under study minus changes in blood flow at the current measurement time relative to the reference measurement time $y_i'^{sum}$ (curve 3), is illustrated in Fig. 4. For clarity, the same plot shows time dependencies of the conductivity that corresponds to the amount of fluid $y_i^{sum}$ in the body tissues in the volume under study (curve 1), and that corresponds to the amount of extracellular fluid in the body tissues in the volume under study $y_i^{out}$ (curve 2) at the current measurement time relative to the reference measurement time (4).

As is seen from the plots, the dependency $y_i'^{sum}$ (curve 3) reflecting the amount of fluid in the body tissues of the volume under study minus blood flow changes observed at the current measurement time does not have any significant ups and downs caused by blood flow changes in the body tissues in the volume under study due to temporary external factors. The latter includes, for example, physical activity or its lack, changes in psychological state, or other factors associated with daily life activities of a human.

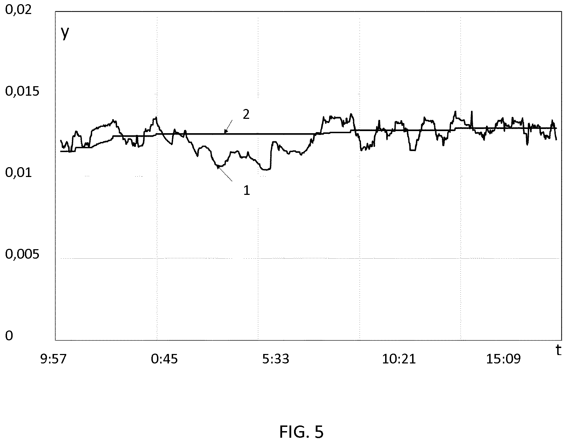

Based on the obtained corrected assessment values $y_i'^{sum}$ proportional to the amount of fluid in the body tissues of the volume under study, the onset of the state of water deficiency in the human body is determined by comparing the above corrected assessment values with a threshold value (Fig. 5).

Here, the threshold value is taken as the corrected assessment value of the conductivity, which is proportional to the amount of fluid in the of body tissues in the volume under study and is associated with the change in blood amount over a given time period taken into account. The threshold value should be less than the maximum value over the entire preceding measurement time by a certain value:

$$y_{thresh}'^{sum} = y_{\max k}'^{sum} - \Delta_{thresh},$$

where:

$y_{thresh}'^{sum}$ - is the threshold value of conductivity,

$y_{\max k}'^{sum}$ - is the maximum value of conductivity in preceding measurements,

$\Delta_{thresh}$ - is a value that determines how smaller the threshold conductivity value must be compared to its maximum value.

It should be noted that the value $y_{\max k}'^{sum}$ within the first few hours of the device use can grow quite high if the method is applied after the user with water deficiency consumed a required amount of water in the process of drinking and/or eating.

The plot (Fig. 5) shows the temporal changes in value $y_i'^{sum}$ (curve 1) and the threshold value, below which the onset of water deficiency in the body occurs (curve 2).

The reliability of determining the onset of water deficiency in a human body increases as the observations grow longer, since the threshold value $y_{\max k}'^{sum}$ will more accurately reflect the maximum amount of water necessary to maintain a particular person's water balance during daily life activities.

The method of the invention is designated to determine current deficiency of water in a healthy human body and may be used to develop various devices or automatic systems for monitoring the state of a human body. The method has been tested on a sufficiently large number of subjects.

**Claims**

1. A method for detecting water deficiency in a human body, the method comprising:

   measuring an impedance value of a portion of the human body at a low frequency and at a high frequency;
   assessing an amount of fluid in body tissues in a volume under study based on an impedance measured at the high frequency at a current measurement time;
   assessing an amount of extracellular fluid in the volume under study based on an impedance value measured at the low frequency at the current measurement time;
   preselecting a reference assessment value of an amount of extracellular fluid in the volume under study at a start of measurements;
   determining a correction value associated with a change of a blood amount in the body tissues in the volume under study at the current measurement time;
   determining a corrected assessment value of the fluid amount in the body tissues in the volume under study, wherein the corrected assessment value is associated with the change of the blood amount in the body tissues in the volume under study at the current measurement time;
   using the corrected assessment value of the fluid amount in the body tissues in the volume under study to determine an onset of a state of water deficiency in the human body.

2. The method of claim 1, further comprising measuring the impedance value of a portion of the human body by using spaced-apart electrodes attached to the human body.

3. The method of claim 1, further comprising using a hematocrit value for determining the corrected assessment value of the fluid amount in the body tissues in the volume under study associated with the change of the blood amount in the body tissues in the volume under study.

4. The method of claim 1, further comprising determining the onset of the state of water deficiency in the human body based on a maximum value of corrected assessment value of the fluid amount in the body tissues in the volume under study associated with the change of the blood amount in the body tissues in the volume under study at the current measurement time.

5. The method of claim 1, further comprising correcting the impedance values of the portion of the human body measured at the low frequency and at the high frequency by using the readings of a body temperature sensor.

6. The method of claim 1, wherein the reference measurement time is preselected succeeding a termination of a transition time necessary to adapt the human body to measurement sensors.

FIG. 1

FIG. 2

FIG. 3

FIG. 4

FIG. 5

## INTERNATIONAL SEARCH REPORT

| | International application No. |
|---|---|
| | PCT/RU 2016/000857 |

**A. CLASSIFICATION OF SUBJECT MATTER**

A61B 5/00 (2006.01); A61B 5/053 (2006.01)

According to International Patent Classification (IPC) or to both national classification and IPC

**B. FIELDS SEARCHED**

Minimum documentation searched (classification system followed by classification symbols)

A61B 5/053, 5/00

Documentation searched other than minimum documentation to the extent that such documents are included in the fields searched

Electronic data base consulted during the international search (name of data base and, where practicable, search terms used)

"Rossiiskaya meditsina", EAPATIS, Esp@cenet, Medline, PAJ, Patentscope, PatSearch (RUPTO internal), Rambler, USPTO, Yandex, Benran, VINITI

**C. DOCUMENTS CONSIDERED TO BE RELEVANT**

| Category* | Citation of document, with indication, where appropriate, of the relevant passages | Relevant to claim No. |
|---|---|---|
| A | RU 2093069 C1 (VOLKOV IURII NIKOLAEVICH et al.) 20.10.1997 | 1-6 |
| A | RU 2251387 C1 (KAPITANOV EVGENII NIKOLAEVICH et al.) 10.05.2005 | 1-6 |
| D, A | RU 2314750 C1 (TSVETKOV ARKADII ALEKSANDROVICH et al.) 20.01.2008 | 1-6 |
| D, A | US 8406865 B2 (COVIDIEN LP) 26.03.2013 | 1-6 |
| D, A | US 5469859 A (N.I.MEDICAL LTD.) 28.11.1995 | 1-6 |
| A | US 2004/0230106 A1 (NELLCOR PURITAN BENNETT INCORPORATED) 18.11.2004 | 1-6 |

| ☐ Further documents are listed in the continuation of Box C. | ☐ See patent family annex. |
|---|---|

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 07 April 2017 (07.04.2017) | 20 April 2017 (20.04.2017) |

| Name and mailing address of the ISA/ | Authorized officer |
|---|---|
| RU | |
| Facsimile No. | Telephone No. |

Form PCT/ISA/210 (second sheet) (July 1998)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5469859 A
- RU 2314750
- US 8406865 B